# EUROPEAN PATENT APPLICATION

(11) **EP 3 547 177 A1**
(43) Date of publication of application: **02.10.2019**
(21) Application number: 17874740.8
(22) Date of filing: 24.11.2017
(51) Int. Cl.: G06F 19/00, G06F 19/12, G06F 19/16, G01N 33/68

(54) **METHOD FOR EXCAVATING NEW DRUG CANDIDATE TARGETING NONSTRUCTURE-STRUCTURE TRANSITION SITE AND APPARATUS FOR EXCAVATING NEW DRUG CANDIDATE**

(30) Priority: 24.11.2016 KR 20160157162
(71) Applicant: Industry - University Cooperation Foundation Hanyang University, 04763 Seoul (KR)
(72) Inventor: NA, Insung, Yangju-si Gyeonggi-do 11513 (KR); KIM, Min Young, Incheon 21521 (KR); UVERSKY, Vladimir, Tampa, Florida 33612 (US); KIM, Chul Geun, Seoul 03981 (KR)
(74) Representative: dompatent von Kreisler Selting Werner - Partnerschaft von Patent- und Rechtsanwälten mbB
(86) International application number: PCT/KR2017/013489
(87) International publication number: WO 2018/097635

(57) **Abstract**

A method of discovering a drug candidate, comprising: a step in which a computer device uses bioinformatics to determine a disorder-to-order transition region of a target protein; a step in which the computer device performs molecular docking on the disorder-to-order transition region in conjunction with a library of specific compounds to select first candidate compounds capable of binding to the disorder-to-order transition region from among the compound library; and a step in which the computer device performs a molecular dynamics simulation for the first candidate compounds and the disorder-to-order transition region to select a second candidate compound from among the first candidate compounds.

## Description

### [Technical Field]

The following description relates to a technique for discovering candidate materials for new drugs.

### [Background Art]

The pharmaceutical industry has recently developed various platforms for developing a new drug. The time and cost required for developing a new drug may be reduced using a platform for discovering a new drug.

The conventional anticancer agents mostly have been based on the regulation for signaling receptors on the surface of cancer cells and phosphorylases/dephosphorylases which are relation with intracellular signaling. However, the conventional anticancer agents may affect normal cells to exhibit severe side effects since the receptors and enzymes are also involved in the survival of normal cells.

Recently, therapeutic agents targeting transcription factors and epigenomes involved in the regulation of gene expression has also been developing. However, since these factors are also required for the maintenance of normal cells *in vivo*, it is not easy to find an effective target.

In the development of an anticancer agent, which has been most actively conducted, the concept of cancer stem cells (CSCs) has newly emerged. The CSCs are present in tumors as well as in normal stem cells, and are known to cause anticancer agent resistance, cancer recurrence and cancer metastasis by maintaining the characteristics of CSCs and creating various differentiated cancer cells through an inherent gene regulatory network. Accordingly, there is a need for developing an anticancer agent capable of controlling new CSCs according to the understanding of a maintenance mechanism of CSCs. There is a need for developing technology capable of inducing differentiation and generation suppression of CSC cells by regulating the plasticity between non-CSC and CSC cells. And the development of this technology may ultimately lead to the development of a new concept target cancer therapeutic agent capable of completely treating cancer.

### [Disclosure]

### [Technical Problem]

In the method of developing a therapeutic agent, a candidate which induces the cell death of cancer cells or differentiation of cancer cells into normal cells is identified by screening a library of natural products or chemical compounds for a specific cell line, or the candidate is identified by confirming binding to/expression of a specific target protein. Since this method may screen various materials, the method has an advantage in that an optimal candidate may be identified, but has a disadvantage in that much time and cost are required.

When a tertiary structure of a target protein is revealed, a candidate capable of binding to a structure of a target site may also be efficiently identified through a computer simulation. However, a predicted effect may not occur when cells/living organisms are treated with the candidate, since the tertiary structures of the target proteins are limitedly revealed *in vivo* and the target protein exhibits a specific activity through interactions with other proteins and post-translational modification.

In addition, it is known that major regulatory proteins do not have a fixed tertiary structure (a disordered region) and the major regulatory proteins exhibits a specific activity by a post-translational modification or interactions with other proteins (Dyson H and Wright PE, Intrinsically unstructured proteins and their function. Nat Rev Mol Cell Biol, 2005, 6: 197). The disordered region is well preserved and binds with another protein to form a specific structure. Accordingly, a method capable of discovering a candidate targeting the disordered region would be very useful for the development of a new drug.

The intrinsically disordered protein (IDP) acts as a dynamic and sensitive switch that forms a stable complex with other proteins or responds to external signals (Uversky VN, (Intrinsically disordered) splice variants in the proteome: implications for novel drug discovery. Genes Genome, 2016, 38: 577). Further, the IDPs exhibit different structures depending on the ambient environment, and also exhibit different functions depending on the cellular environment (Uversky VN and Dunker AK, Understanding protein non-folding. Biochim Biophys Acta, 2010, 1804: 1231). Accordingly, it is difficult to derive a candidate targeting the IDP by using a conventional computer simulation.

Furthermore, when a discovered candidate is a small compound, an unexpected side effect may also be exhibited by binding to other non-target proteins. As a result, it is estimated that 650,000 protein-protein interactions appear in vivo, but there is only one compound that has been approved for clinical trials for inhibiting a protein-protein interaction (Douglas R. Green, A BH3 Mimetic for Killing Cancer Cells. Cell, 2016, 165: 1560).

The following description is intended to provide a technique or platform for discovering a new candidate that suppresses or activates expression in a specific protein based on a disorder-to-order transition region.

### [Technical Solution]

In one general aspect, there is provided a method of discovering a new drug candidate targeting a disorder-to-order transition region, comprises: a step in which a computer device uses bioinformatics to determine a disorder-to-order transition region of a target protein; a step in which the computer device performs molecular docking on the disorder-to-order transition region in conjunction with a library of specific compounds to select first candidate compounds capable of binding to the disorder-to-order transition region from among the compound library; and a step in which the computer device performs a molecular dynamics simulation for the first candidate compounds and the disorder-to-order transition region to select a second candidate compound from among the first candidate compounds. Furthermore, a method of discovering a new drug candidate targeting a disorder-to-order transition region may further comprise: a step of verifying whether the second candidate compound also binds to other candidate proteins.

### [Advantageous Effects]

The following description may derive a new drug candidate very quickly without much experimentation. Furthermore, the following description may dramatically reduce the cost and time required for developing a new drug by excluding a material that is expected to have side effects in advance.

### [Description of Drawings]

FIG. 1 illustrates an example of the results of identifying a disorder-to-order transition region for MBD2 and p66α.
FIG. 2 illustrates an example of the results of performing a molecular dynamics simulation on a target protein MBD2 and a candidate compound.
FIG. 3 illustrates an example in which molecular docking and molecular dynamics analysis are performed on p66α with respect to the identified candidate compound.
FIG. 4 illustrates resulting data for the structures of two candidate compounds and the ability to suppress the interaction of MBD2 with p66α by a co-immuoprecipitation assay (Co-IP).
FIG. 5 illustrates resulting data of a Luc reporter assay showing the effect of the treatment with two candidate compounds on the role of MBD2 or p66α in the expression of a target gene of a CP2c transcriptional factor complex.
FIG. 6 illustrates an example of the flowchart of a method of discovering a new drug candidate targeting a disorder-to-order transition region.
FIG. 7 illustrates an example of the flowchart of a process for performing verification of a discovered candidate.
FIG. 8 illustrates an example of an apparatus for discovering a new drug candidate targeting a disorder-to-order transition region.

### [Modes of the Invention]

As described above, a nonstructural site of a protein does not have a stereotypical structure. Accordingly, the nonstructural site of the protein is not utilized for identification of a candidate compound using a computer in the related art.

However, the technology to be described below identifies a candidate compound by performing molecular docking and a molecular dynamics simulation for a region where a disorder-to-order transition is expected to occur due to protein interactions. Furthermore, the technology to be described below may confirm whether an identified candidate compound has any side effect of binding to other proteins in advance. Further, through experiments, it was confirmed that the candidate compound identified by the technology to be described below exhibited efficacy in cultured cell line and animal models.

In a protein structure, a disorder (non-structure) region refers to a part in which the structure is not stereotyped, and an order (structure) region refers to a part in which a biochemical reaction occurs and the structure is stereotyped.

The candidate compound refers to a material which binds to a specific site of a specific protein to inhibit or activate the activity of the corresponding protein. The candidate compound may be a new drug candidate for a specific disease.

A computer device performs a discovery of a new drug candidate to be described below. The computer device includes a terminal device such as a personal computer (PC), a notebook-sized computer, and a smart machine. Furthermore, the computer device also includes an object on a network such as an analysis server. In the latter case, a user performs a process of identifying a new drug candidate by accessing a server via a client device. An apparatus or system for discovering a new drug candidate will be described below.

The technology to be described below demonstrated identification and effects of a candidate through specific experiments. Hereinafter, an MBD2-p66α (GATAD2A) interaction site will be mainly described. A chromatin remodeling complex (CRC) Mi-2/NuRD is important even for the survival of normal cells, but plays an important role even in cancer cells. The MBD2-p66α (GATAD2A) interaction site is important for the maintenance of the structure of Mi-2/NuRD. In the following description, a process of identifying a candidate compound by performing molecular docking and a molecular dynamics simulation for the MBD2-p66α (GATAD2A) interaction site will be described. Here, the candidate compound is a material which inhibits the formation of an intact complex by interfering with the MBD2-p66α interaction.

First, a target protein for describing the process of identifying a new drug candidate will be described. Of course, the specific target protein to be described is only an example.

### Selection of Target Protein

Cancer cells in a tumor are not identical to one another, and it has been reported that these various cancer cells originate from cancer stem cells (CSCs), and a chromatin remodeling complex (CRC) is also associated with metastasis and recurrence of cancer. Accordingly, the formation of tumors is ultimately attributed to mutations of an oncogene and a tumor suppressor, but the epigenetic regulatory mechanisms of the CSCs (DNA methylation, nucleosome reconfiguration, and histone formulation) act importantly on the origin of a tumor and the maintenance and metastasis of the tumor.

Prior studies have reported that when the activity of the epigenetic regulatory mechanism is suppressed, metastasis and recurrence of cancer may be efficiently suppressed because it is possible to control the self-reproduction of CSCs and the appearance of various differentiated cancer cells. Accordingly, various types of DNA methyltransferase suppressors and histone deacetylase (HDACs) suppressors have been developed and clinical experiments have been performed, but these suppressors may efficiently control cancer cells, but have problems in that these suppressors affect normal cells and exhibit severe toxicity.

For example, DNA methylation in a promoter results in the suppression of expression of a target gene while maintaining a close relationship with chromatin and nucleosomes. Various protein complexes exhibiting interactions are involved in the DNA methylation process of the promoter. HDACs responsible for deacetylation of histone proteins, which are essential for the suppression of expression of a gene, are also always involved in the suppression of expression of the target gene by DNA methylation. However, HDACs are members of various transcriptional suppression complexes. Accordingly, an anticancer agent controlling the same cannot help but exhibit side effects.

Accordingly, an ideal anticancer agent based on epigenetics is a member of the transcriptional suppression complex, and should also target a member having cancer cell specificity without being essential to the function of normal cells.

A Mi-2/NuRD chromatin remodeling complex recognizes and binds to a methylated DNA region to suppress transcription. Further, the Mi-2/NuRD chromatin remodeling complex also includes a HDAC as a member. Since the Mi-2/NuRD complex may directly bind to a DNA methylation site and a DNA methylation enzyme, the Mi-2/NuRD complex performs a very important function in the epigenetic suppression of expression of a gene.

In an experiment for identifying a drug candidate, MBD2 among Mi-2/NuRD CRC constituent proteins was set as a target. MBD2 has characteristics as follows.
1) MBD2 gene knockout mice exhibit normal survival and reproduction without exhibiting significantly deleterious effects (Hendrich B, Guy J, Ramsahoye B, Wilson VA, Bird A, Closely related proteins MBD2 and MBD3 play distinctive but interacting roles in mouse development. Genes Dev, 2001, 15: 710).
2) When the expression of MBD2 is reduced in cancer cell lines and cancer xenograft animal models, a growth inhibitory effect on cancer is exhibited (Slack A, Bovenzi V, Bigey P, Ivanov MA, Ramchandani S, Bhattacharya S, tenOever B, Lamrihi B, Scherman D, Szyf M, Antisense MBD2 gene therapy inhibits tumorigenesis. J Gene Med, 2002, 4: 381; Sansom OJ, Berger J, Bishop SM, Hendrich B, Bird A, Clarke AR, Deficiency of Mbd2 suppresses intestinal tumorigenesis. Nat Genet, 2003, 34: 145; Mian OY, Wang SZ, Zhu SZ, Gnanapragasam MN, Graham L, Bear HD, Ginder GD, Methyl-binding domain protein 2-dependent proliferation and survival of breast cancer cells. Mol Cancer Res, 2011, 9: 1152).

While the transcription regulatory mechanism of the globin gene by a transcriptional factor CP2c (also called TFCP2, LSF, LBP1, and USF) in a murine erythroleukemia (MEL) cell line model was studied, it was confirmed that the reduction in expression of MBD2 is essential for a normal erythroid differentiation process. The MEL cell line is a cancer cell whose differentiation is suspended in a proerythroblast state during the erythroid differentiation process, but exhibits the expression of the globin gene along with terminal differentiation when the culture solution is treated with chemical inducers, such as dimethyl sulfoxide (DMSO) or hexamethylene bisacetamide (HMBA).

Further, it was confirmed that CP2c formed a CBP complex with CP2b and PIAS1 proteins, and was involved in the erythroid specific globin gene transcription (Kang HC, Chae JH, Lee YH, Park MA, Shin JH, Kim SH, Ye SK, Cho YS, Fiering S, Kim CG, Erythroid cell-specific alpha-globin gene regulation by the CP2 transcription factor family. Mol Cell Biol, 2005, 25: 6005; Kang HC, Chae JH, Jeon J, Kim W, Ha DH, Shin JH, Kim CG, Kim CG, PIAS1 regulates CP2c localization and active promoter complex formation in erythroid cell-specific alpha-globin expression. Nucleic Acids Res, 2010, 38; 5456).

In addition, through a yeast two hybrid assay method, it was revealed that p66α (GATAD2A), which is one of the Mi-2/NuRD CRC members, directly binds to CP2c (Kang HC, Chung BM, Chae JH, Yang SI, Kim CG, Kim CG, Identification and characterization of four novel peptide motifs that recognize distinct regions of the transcription factor CP2. FEBS J, 2005, 272: 1265).

The transcriptional activity of CBP transcriptional factor complexes (CP2c, CP2b, and PIAS1) is suppressed through p66α binding to CP2c. And the splenomegaly and the tumorigenesis in blood, a spleen and a liver are were remarkably suppressed in normal control cells, when a MEL cell line in which the expression of p66α was reduced was intravenously injected into immunodeficient mice. The expression of p66α was constantly maintained during the induction of MEL cell erythroid differentiation and during the erythroid differentiation process in bone marrow, whereas the expression of MBD2 known to directly bind to p66α as another member of Mi-2/NuRD CRC was drastically decreased.

The transcriptional activity of the actual CBP complex exhibited an inversely proportional relationship with the expression of MBD2, and a MEL cell line in which the expression of MBD2 was reduced exhibited spontaneous erythroid differentiation. Furthermore, the MBD2-p66α interaction was confirmed that was involved in the activity of the CBP complex. Mi-2/NuRD CRC in undifferentiated MEL cells was confirmed that Mi-2/NuRD CRC with MBD2 suppressed the expression of a target gene as a typical CRC (restrictive Mi-2/NuRD CRC), whereas Mi-2/NuRD CRC without MBD2 aided the transcriptional activity of the CBP complex in a state where the Mi-2/NuRD CRC is not separated from a globin gene promoter during the normal erythroid differentiation.

From the above-described experimental results, it may be estimated that MBD2 does not affect the survival of normal cells, and the MBD2-p66α interaction is important for the function of suppressing the gene expression of Mi-2/NuRD CRC. Hereinafter, a process of discovering a candidate which may be an anticancer agent focused on the MBD2-p66α interaction site will be described under the premise that the MBD2-p66α interaction is important for the function of suppressing the gene expression of Mi-2/NuRD CRC. That is, hereinafter, the description will be described focused on an interaction protein which is MBD2-p66α. However, when a protein involved in a specific disease is specified, the technology to be described below can be universally applied to a process of finding a candidate which binds to a specific site of the corresponding protein to suppress or activate gene expression.

Hereinafter, a process of identifying a new drug candidate based on the above-described target protein MBD2 (MBD2-p66α) will be described.

### Discovery of Therapeutic Agent Candidate Targeting MBD2-p66α Interaction Site Based on Molecular Docking and Molecular Dynamics Simulation

(1) As a first step, a disorder-to-order transition region is identified from the structure of a target protein. Through bioinformatics techniques including intrinsic disorder prediction (IDP), sequence alignment, and structural alignment for MBD2 and p66α, a disorder-to-order transition region between MBD2 and p66α is identified.

According to the necessity of disorder region prediction, conventional studies have suggested methods of using sequence data already known rather than the structural data of a protein, and methods of predicting a region through classification/prediction algorithms by extracting a feature exhibiting specific properties of a protein. The existing classification/prediction technique for predicting a disorder region from the sequence data of the protein usually uses patterns generated using a method of applying a sliding window to sequence data and using feature selection. Then, these patterns are input to a classification/prediction model to predict a disorder region. The classification/prediction model is usually one of a support vector machine (SVM), neural networks, regression, and the like.

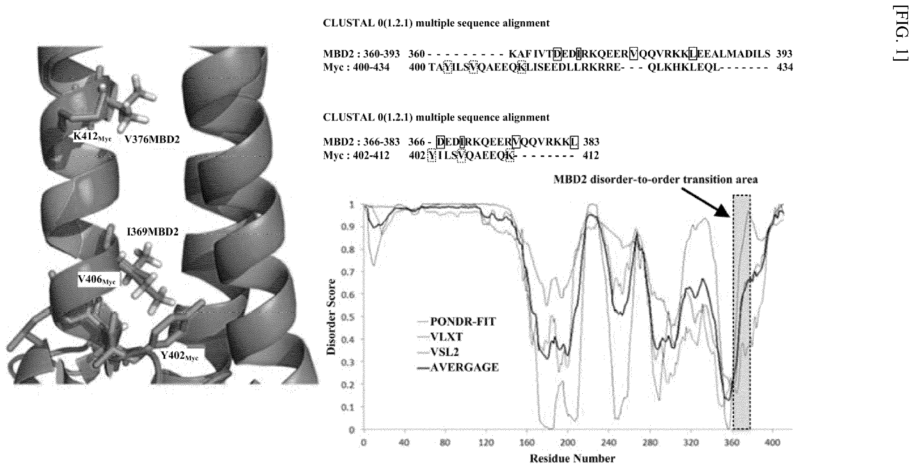

FIG. 1 illustrates an example of the results of identifying a disorder-to-order transition region for MBD2 and p66α. FIG. 1 corresponds to a result of applying a prediction model for finding a disorder region based on structural information on MBD2 and p66α. FIG. 1 is an example in which a disorder region is found using PONDR-FIT, PONDR-VLXT, and PONDR-VSL2. A disorder score for each constituent amino acid was predicted by inputting the amino acid sequence of each protein to PONDR-FIT, PONDR-VLXT, and PONDR-VSL2. Further, for three disorder score results, an average value was measured as an amino acid unit. As a result of graphing the average value according to the amino acid sequence, an interval at which about 40 amino acids were continuously increased commonly appeared at a site interacting with each partner protein. Meanwhile, it was revealed that in MBD2, the N-terminal in this region could form a binding structure with each interacting protein. In FIG. 1, a region marked with a dotted rectangle corresponds to a disorder-to-order transition region between MBD2 and p66α.

It could be confirmed that a portion of the disorder-to-order transition region for MBD2 coincided with an interaction site of conventionally known MBD2 and p66α (Gnanapragasam MN, Scarsdale JN, Amaya ML, Webb HD, Desai MA, Walavalkar NM, Wang SZ, Zu Zhu S, Ginder GD, Williams DC Jr, p66Alpha-MBD2 coiled-coil interaction and recruitment of Mi-2 are critical for globin gene silencing by the MBD2-NuRD complex. Proc Natl Acad Sci USA, 2011, 108: 7487; Walavalkar NM, Gordon N, Williams DC Jr, Unique features of the anti-parallel, heterodimeric coiled-coil interaction between methyl-cytosine binding domain 2 (MBD2) homologues and GATA zinc finger domain containing 2A (GATAD2A/p66α). J Biol Chem, 2013, 288: 3419). That is, it can be said that the disorder-to-order transition region corresponds to a major site interacting with another protein in a specific protein.

Hereinafter, a discovery method for a candidate binding to a target site (disorder-to-order transition region) of a target protein will be described.

The discovery method uses a molecular docking and a molecular dynamics simulation for search a candidate capable of binding to a target site. The basis for the discovery method is as follows.

A Myc target compound 10058-F4 was first identified as a drug targeting a disorder region. However, the Myc target compound 10058-F4 was not found based on a computer simulation, but was discovered by using a yeast two-hybrid system (Yin X, Giap C, Lazo JS, Prochownik EV, Low molecular weight inhibitors of Myc-Max interaction and function. Oncogene, 2003, 22(40) 6151-9). Through a subsequent biochemical analysis, it was revealed that 10058-F4 specifically binds to a 402-412 region of Myc (Follis AV, Hammoudeh DI, Wang H, Prochownik EV, Metallo SJ, Structural rationale for the coupled binding and unfolding of the c-Myc oncoprotein by small molecules. Chemistry & Biology, 2008, 15(11) 1149-55). Further, a molecular dynamics simulation for analyzing binding characteristics of 10058-F4 within this region was performed, and as a result, the highest number of interactions were observed at Y402 and K412 (Michel J and Cuchillo R, The impact of small molecule binding on the energy landscape of the intrinsically disordered protein C-myc. PLoS One, 2012, 7: e41070). In the case of Myc, the site is accepted by academia as a disorder-to-order transition region exhibiting structural variation characteristics according to protein interactions (Uversky VN, Intrinsically disordered proteins and novel strategies for drug discovery. Expert Opinion on Drug Discovery, 2012, 7: 475).

The technology to be described below uses a platform for finding and analyzing a new compound which is expected to inhibit interactions between MBD2 and p66α by reversely utilizing a method of performing molecular docking and a molecular dynamics simulation, which reveals binding characteristics between 10058-F4 and Myc.

Meanwhile, Myc also has disorder-to-order transition region characteristics at a binding site with a protein exhibiting an interaction, like MBD2. In addition, an interval at which about 40 amino acids were continuously increased occurred in MBD2, and it was revealed that the N-terminal of this region could form a binding structure with each interaction protein in both Myc and MBD2 (Nair SK, Burley SK, X-ray structures of Myc-Max and Mad-Max recognizing DNA. Molecular bases of regulation by proto-oncogenic transcription factors. Cell, 2003, 112(2) 193-205; Gnanapragasam MN, Scarsdale JN, Amaya ML, Webb HD, Desai MA, Walavalkar NM, Wang SZ, Zu Zhu S, Ginder GD, Williams DC Jr, p66Alpha-MBD2 coiled-coil interaction and recruitment of Mi-2 are critical for globin gene silencing by the MBD2-NuRD complex. Proc Natl Acad Sci USA, 2011, 108: 7487).
(2) As a second step, a candidate capable of binding to the identified disorder-to-order transition region is found. Molecular docking is performed on the identified disorder-to-order transition region using a specific compound library. A candidate compound capable of binding to the disorder-to-order transition region identified through the performed molecular docking is determined.
   In an experiment in which the candidate compound is determined, molecular docking was performed using a DOCK program in conjunction with a ZINC compound library for the MBD2 structure. As a result of performing molecular docking using the DOCK program in conjunction with the ZINC library, 1,000 candidate compounds could be derived. The DOCK program is a program capable of confirming whether a protein binds to a ligand. A candidate compound capable of binding to the disorder-to-order transition region of MBD2 in the ZINC compound library may be determined using any one of various DOCK programs.
(3) As a third step, a final candidate compound is derived by performing a molecular dynamics simulation on the candidate compound. For example, the molecular dynamics simulation may be performed/analyzed by utilizing a Gromacs program.

Molecular dynamics simulations were performed on 1,000 candidate compounds derived as a result of performing molecular docking by using the DOCK in conjunction with the ZINC library. The top two compounds with the highest binding energy value for the target site (disorder-to-order transition region) of the protein would be derived as the final candidate compounds by performing the molecular dynamics simulation. The final candidate compounds were ZINC40430779 and ZINC60177071 which are capable of forming three hydrogen bonds with MBD2.

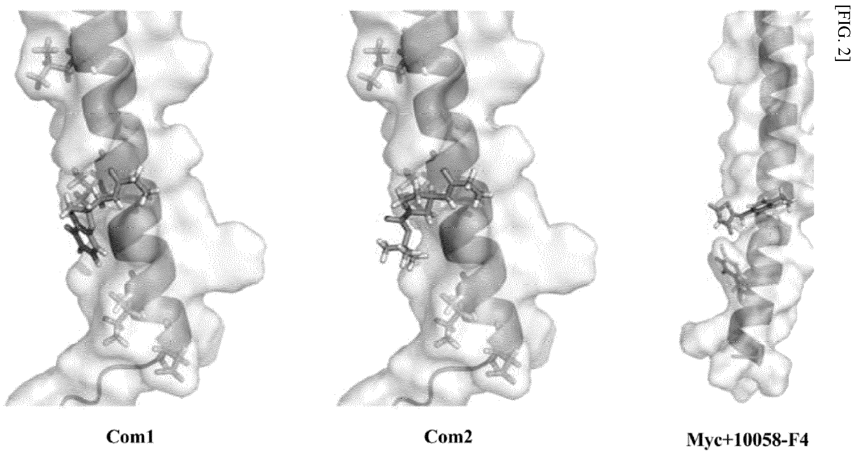

After the molecular dynamics simulation of three sets (Com1 = 'MBD2+ZINC40430779', Com2 = 'MBD2+ZINC60177071', Myc+10058-F4) using the Gromacs program, a protein-compound contact heatmap was derived based on the number of contacts between the amino acids of the protein and the compound. FIG. 2 illustrates an example of the results of performing a molecular dynamics simulation on a target protein MBD2 and a candidate compound. Myc+10058-F4 was illustrated in order to explain a control for MBD2.

As a result of performing the molecular dynamics simulation, it was confirmed that D368 of MBD2 binds closest to ZINC40430779, whereas Q372 of MBD2 binds close to ZINC60177071. The interaction energy coincided with the contact number pattern, and it was confirmed that the two compounds bind at an angle different from D368 of MBD2.

In order to verify a structural variation occurring when MBD2 alone and a MBD2-p66α complex bind to the compound, backbone torsion angles of amino acids involved in the bond to p66α were obtained and a T-test analysis was conducted to see where there is a difference. As a result of the analysis, a significant difference in the backbone torsion angle of the amino acid was exhibited.
[com1, ZINC40430779; #086567 (Fluorochem)] 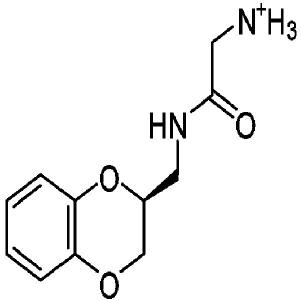
[com2, ZINC60177071; #080579 (Fluorochem)]

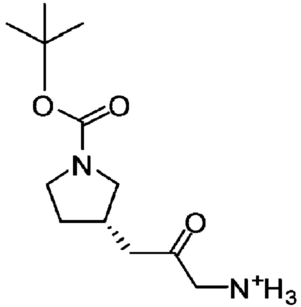

Through the above-described process, a candidate suppressing the gene expression of Mi-2/NuRD CRC was derived by binding MBD2 to a disorder-to-order transition region which is an interaction site of p66α. Meanwhile, the derived final candidate compound is also likely to bring about side effects by binding to another protein other than a target protein. Therefore, it is preferred to verify whether the final candidate compound binds to another protein.

### Verification of Possibility of Therapeutic Agent Candidate Binding to and Interacting with Another Protein Based on Molecular Docking and Molecular Dynamics Simulation

Nonstructural site properties of a protein are determined at an amino acid unit, and the structure of a protein is determined by the configuration of an amino acid. Accordingly, it is possible to predict a region in which a candidate can binds to a protein structure through the nonstructural site. The region in which a candidate is highly likely to bind to another material is called a molecular recognition feature (MoRF). Although it is possible to infer the possibility that the corresponding protein binds to another material through a MoRF prediction algorithm, there is also a case where the possibility may be found by searching a database including protein structural information as in MBD2 and Myc, so that a new algorithm may be developed in consideration of these possibilities.

First, several amino acid sequences of proteins determined to likely bind to the derived final candidate compound are configured as a set. In theory, the amino acid sequences for all obtainable proteins may also be prepared. A protein likely to bind to the final candidate compound is called a binding candidate protein.
(i) An amino acid sequence of a binding candidate protein is input to a program (or algorithm) for predicting a disorder region. An algorithm such as the above-described PONDR-FIT, PONDR-VLXT, and PONDR-VSL2 may be used. A disorder region of a binding candidate protein is predicted by using the algorithm.
(ii) Further, the possibility is predicted by using an algorithm such as ANCHOR and MoRFpred, which predicts a binding region such as a MoRF. That is, a candidate region which is likely to bind to a final candidate compound among the amino acid sequences of the binding candidate proteins is determined.
(iii) When a candidate region is determined among the amino acid sequences, a protein structure may be estimate based on the candidate region.
   When a candidate region is specified, the possibility of binding to another protein would be confirmed based on a database including bioinformatics information. And it would be also confirmed whether the structure of the candidate region is present in a Protein Data Bank (PDB). When the sequence is present in the PDB, a structure of the candidate region would be identified from the PDB.
   If the structure is not present, it is determined whether the region is a MoRF. If the region is a MoRF, the total protein structure is obtained through homology modeling. If the region is not a MoRF, but a disorder-to-order transition region, a protein structure of the corresponding disorder-to-order transition region is obtained through homology modeling. Homology modeling is a technique for estimating a protein structure of the corresponding sequence based on the amino acid sequence.
(iv) When the structure of the binding candidate protein is estimated, it is confirmed whether or the degree to which the binding candidate protein binds to or interacts with a final candidate compound. For this purpose, a protein highly likely to bind to a therapeutic agent candidate is finally selected by performing molecular docking (using DOCK, Autodock, Autodock vina, and the like) on the binding candidate protein and the final candidate compound, and performing a molecular dynamics simulation (using Gromacs, AMBER, CHARMM, OpenMM, and the like) on a complex structure subjected to molecular docking.
(v) Finally, a candidate compound, which is far better than the binding candidate protein in terms of binding to and interaction with the target protein (MDB2 in the above-described experiment) among the final candidate compounds, is selected as a final compound. The final compound may be used as a candidate for developing a new drug.

Since a candidate compound capable of exhibiting additional effects including side effects may be excluded in advance through the above-described verification process, a clinically applicable therapeutic agent may be efficiently identified. Accordingly, it is possible to reduce the expenditure of a great deal of time and money in the process of developing a therapeutic agent.

For the two compounds (com1, ZINC40430779 and com2, ZINC60177071) found to target the above-described MBD2, the MBD2 binding amino acid of p66α was selected as a target point, and then the number of contacts was analyzed by performing molecular docking and a molecular dynamics simulation.

Molecular docking using DOCK was performed by conjugating ZINC40430779 and ZINC60177071 as a compound library, employing p66α as a target protein, and employing 4 amino acids (1145, L152, L159, and R166) of p66α known to bind to MBD2 as target points. Among them, binding complex structures having excellent binding energy values were selected, and the interaction of p66α with ZINC40430779 or ZINC60177071 was analyzed by performing a molecular dynamics simulation on the binding complex structures.

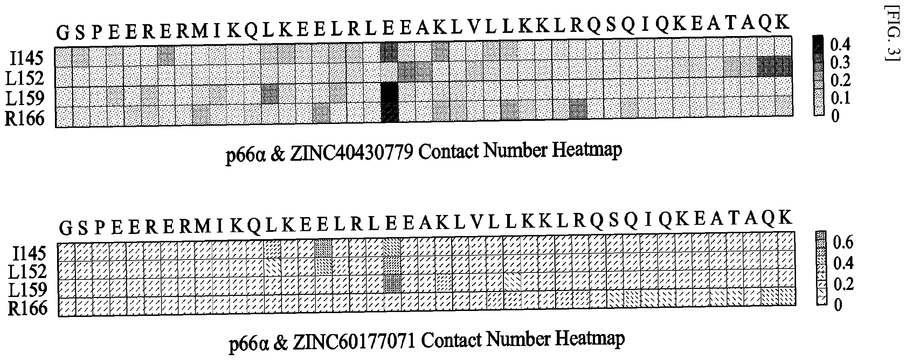

FIG. 3 illustrates an example in which molecular docking and molecular dynamics analysis are performed on p66α with respect to the identified candidate compound. A molecular dynamics simulation was performed on the respective four sites (1145, L152, L159, and R166) for ZINC40430779 and ZINC60177071 by using the Gromacs program. As a result of deriving a protein-compound contact heatmap from 5 sets based on the number of contacts between the amino acids of the protein and the compound after performing a molecular dynamics simulation, E155 of p66α relatively most frequently contacted both compounds in each set.

As a result of comparing molecular dynamics sets based on the two compounds, three types (I145com1, L159com1, and R166com1) among the four sets to which ZINC40430779 binds exhibited a high level of contact number density with E155, and one type (L152com1) exhibited a relatively high contact number density with E156. In contrast, two types (L152com2 and L159com2) among the four sets binding to ZINC60177071 exhibited a high level of contact number density with E155, and one type (I145com2) exhibited a high level of contact number density with E151. However, one type (R166com2) did not exhibit a specific contact number density with any amino acid.

Accordingly, although both ZINC40430779 and ZINC60177071 target the disorder-to-order transition region of MBD2, as a result of performing a molecular dynamic simulation on the possibility of binding to four amino acids (1145, L152, L159, and R166) of p66α, it could be predicted that ZINC40430779 could show better binding in an E155-E156 region of p66α than ZINC60177071. Ultimately, ZINC40430779 may be selected as a final compound. Of course, both compounds may also be selected as the final compound in some cases.

Finally, the MBD2-p66α interaction inhibition ability of the two compounds (ZINC40430779 and ZINC60177071) which have been mentioned as a new drug candidate was confirmed through an experiment.

### Confirmation of MBD2-p66α Interaction Inhibition for Two Candidates

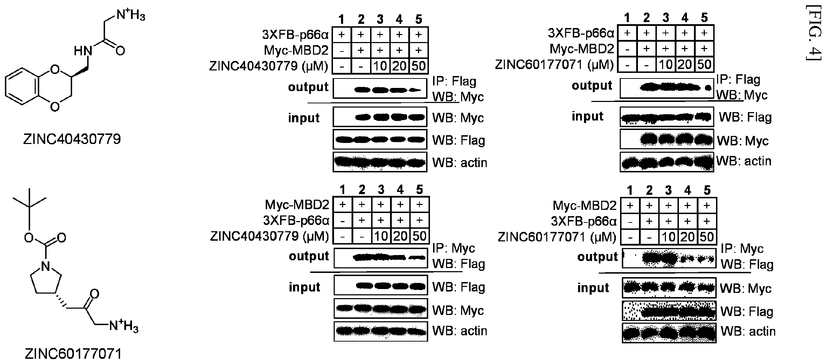

It was analyzed whether the two compounds selected above could actually suppress the interaction of MBD2 with p66α by a co-immunoprecipitation assay (Co-IP). FIG. 4 illustrates resulting data for the structures of two candidate compounds and the ability to suppress the interaction of MBD2 with p66α by a co-immuoprecipitation assay (Co-IP).

As a result of performing Co-IP after treating a cell extract obtained by transducing a 293T cell line with MBD2 and p66α protein overexpression vectors marked with 3XFB and Myc, respectively, it was confirmed that both the candidate compounds suppressed the binding of MBD2 and p66α in a concentration dependent manner.

The researchers of the drug discovery technique in this description were confirmed in a previous study that the expression of a target gene by a CP2c transcriptional factor complex is suppressed by the overexpression of not only intact Mi-2/NuRD CRC, but also MBD2 or p66α, whereas the expression of the target gene is restored by the reduction in expression of MBD2 or p66α or the suppression of the interaction of MBD2 with p66α.

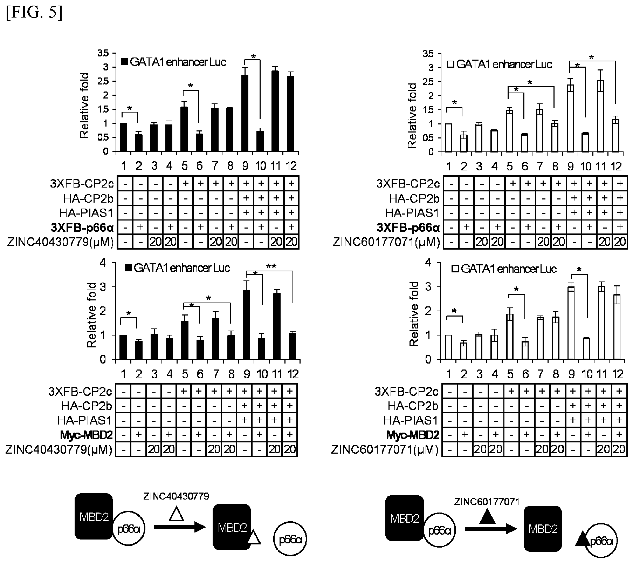

Accordingly, it was analyzed by a Luc reporter assay whether the reduction in expression of the target gene of the CP2c transcriptional factor complex by the overexpression of MBD2 or p66α was restored by treatment with a compound for suppressing the interaction of MBD2 and p66α selected above. FIG. 5 illustrates resulting data from a Luc reporter assay showing the effect of the treatment with two candidate compounds on the role of MBD2 or p66α in the target gene expression of a CP2c transcriptional factor complex.

In this case, a GATA1 enhancer was used as a CP2c transcriptional factor target sequence which controls the expression of the Luc reporter gene, and an analysis was performed by transducing 293T cells with various combinations of CBP complex protein overexpression vectors. As a result, the compound ZINC40430779 (#086567, Fluorochem) restored the suppression of the expression of the target gene by the overexpression of p66α, but failed to restore the suppression of the expression of the target gene by the overexpression of MBD2, and the compound ZINC60177071 (#080579, Fluorochem) exhibited an effect opposite to the effect of the compound ZINC40430779.

Accordingly, it could be inferred and confirmed that the compound ZINC40430779 binds to p66α to suppress the interaction with MBD2, whereas the compound ZINC60177071 binds to MBD2 to suppress the interaction with p66α. This is a result coinciding with a result predicted from a result (FIG. 3) of selecting the MBD2 binding amino acid of p66α as a target point, and then analyzing the number of contacts by performing molecular docking and a molecular dynamics simulation for the two compounds (com1, ZINC40430779 and com2, ZINC60177071) found to target the above-described MBD2.

In conclusion, these verification results and experimental data show that the above-described algorithm or platform for identifying a new drug candidate may be very useful for discovering a new drug.

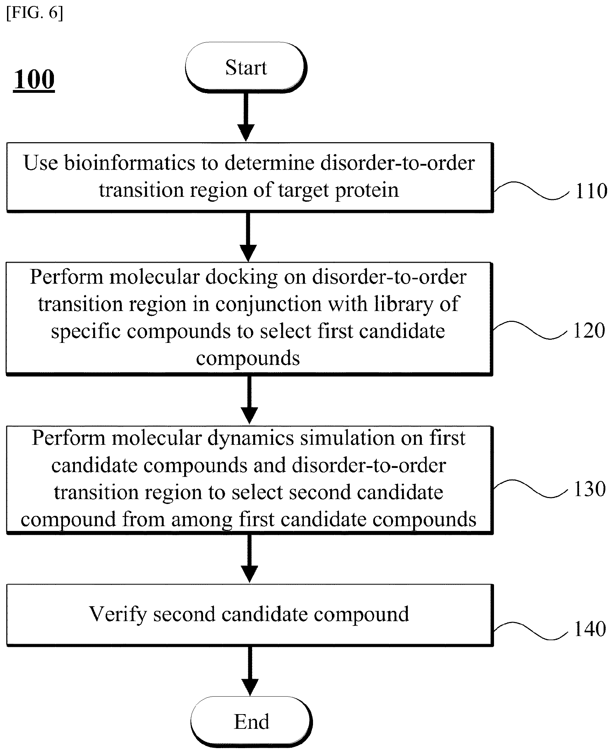

The above-described process of discovering a new drug candidate will be summarized. A computer device performs a process of discovering a new drug candidate. FIG. 6 illustrates an example of the flowchart of a method 100 for discovering a new drug candidate targeting a disorder-to-order transition region.

The computer device predicts a disorder region of a target protein (110). For example, the computer device can predict a disorder-to-order transition region by measuring a disorder score of a protein using a program (web algorithm) such as PONDR-FIT, PONDR-VLXT, and PONDR-VSL. The target protein is a material having activity on a pathway for a specific disease. As described above, the technique for discovering a new drug candidate is not limited to a specific disease or a specific target protein. The candidate compound corresponds to a material which binds to a target protein to suppress or activate the target protein, such that another material ultimately does not bind to the target protein. The computer device uses bioinformatics information already known for the target protein to determine a disorder-to-order transition region of the target protein.

Thereafter, the computer device performs molecular docking on a disorder-to-order transition region of the determined target protein in conjunction with a library of specific compounds (120). For example, the computer device may use a molecular docking program (DOCK, AutoDock, AutoDock vina, and the like) to perform molecular docking on a disorder-to-order transition region and a specific compound. The library of specific compounds includes information on the structures of a plurality of compounds. The library of specific compounds may be a type including a specific molecule. In some cases, the compound library may also include information on the structures of all compounds found so far. The compound library may be possessed in advance by a computer device, or may be received and acquired by a database located in a network. Molecular docking may utilize the above-described various docking programs. The computer device performs molecular docking on a disorder-to-order transition region and a specific compound to select first candidate compounds capable of binding to the disorder-to-order transition region (120). In this case, the computer device may predict a structure for a disorder-to-order transition region, and may select compounds capable of entering the corresponding structure as the first candidate compounds. In some cases, the computer device may also set a specific reference and compare a separation distance between a disorder-to-order transition region and a compound binding to the corresponding site with a critical value to select compounds having a separation distance within the critical value as the first candidate compounds.

The compute device performs a molecular dynamics simulation on the first candidate compounds and the disorder-to-order transition region (130). For example, the computer device may perform the molecular dynamics simulation by executing a program such as Gromacs, AMBER, CHARMM, and OpenMM. In this case, the computer device analyzes the simulation result to select a second candidate compound that is more suitable from among the first candidate compounds. For example, the computer device may select, as a second candidate compound, a compound having a better binding strength with the disorder-to-order transition region from among the first candidate compounds. Further, for example, as a result of the molecular dynamics simulation, the computer device may select, as a second candidate compound, a compound having a higher number of sites binding to the disorder-to-order transition region or higher binding energy than the reference values from among the first candidate compounds.

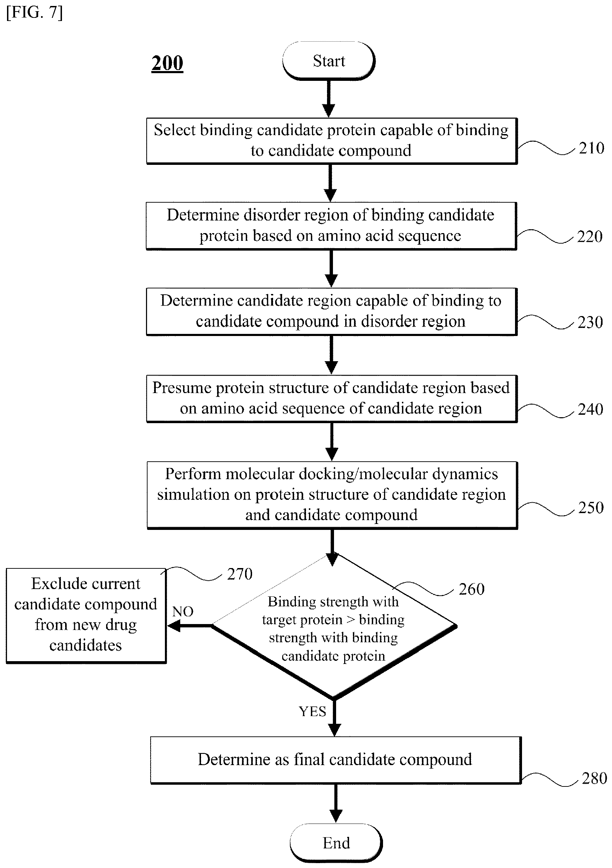

The computer device may determine the second candidate compound as a candidate for developing a new drug. Furthermore, the computer may verify whether the second candidate compound also binds to a protein other than the target protein (140). The verification process will be described in FIG. 7.

FIG. 7 illustrates an example of the flowchart of a process 200 for performing the verification on a discovered candidate. First, the computer device selects another binding candidate protein to which the derived candidate protein (above-described second compound) can bind (210). A plurality of binding candidate proteins are preferred. When there is a protein group known to be capable of binding to a candidate compound in advance, the corresponding protein group may be selected as a binding candidate protein. When there is no information known in advance, the computer device may also select all proteins whose structures have been known until now as a binding candidate protein in some cases.

The computer device determines a disorder region of the binding candidate protein based on an amino acid sequence of the binding candidate protein (220). The computer device may use the above-described disorder region prediction algorithm to determine a disorder region of the binding candidate protein. The amino acid sequence of the binding candidate protein may be information stored in a separate database.

The computer device may use an algorithm such as ANCHOR and MoRFpred, which predicts a binding region such as a MoRF for the disorder region of the binding candidate protein to predict the binding possibility (230). The computer device predicts the possibility that the binding candidate protein binds to the disorder region in advance to determine a region which is likely to have a value equal to or greater than a predetermined reference value as a candidate region (230).

The computer device estimates a protein structure of a candidate region based on the amino acid sequence of the candidate region (240). The computer device confirms whether the amino acid sequence of the candidate region is present in the Protein Data Bank (PDB). When the sequence is present in the PDB, an expected structure of the corresponding protein is obtained from the PDB. If the structure is not present, the computer determines whether the region is a MoRF. If the region is a MoRF, the total protein structure is obtained through homology modeling. If the region is a disorder-to-order transition region rather than a MoRF, the computer device obtains a protein structure of the corresponding disorder-to-order transition region through homology modeling.

Now, the computer device performs molecular docking and a molecular dynamics simulation on a protein structure of a candidate region and the candidate compound (250). The molecular docking and molecular dynamics simulation use the related programs described above. The molecular docking and molecular dynamics simulation results exhibit the degree to which the protein structure of the candidate region binds to the candidate compound.

The computer device compares a binding strength (second binding strength) of a protein structure of a candidate region to a candidate compound with a binding strength (first binding strength) of a target protein to the candidate compound. When the first binding strength is much higher than the second binding strength, the computer device may determine a candidate compound (second candidate compound) as a final compound (280). Further, when the first binding strength is higher than the second binding strength by a reference value or more, the computer device may determine a candidate compound (second candidate compound) as a final compound. In addition, when the first binding strength is higher than the second binding strength, the computer device may determine a candidate compound (second candidate compound) as a final compound.

If the first binding strength is lower than the second binding strength or has a binding strength similar to the second binding strength within the critical range, a current candidate compound (second candidate compound) may be excluded from the new drug candidates (270).

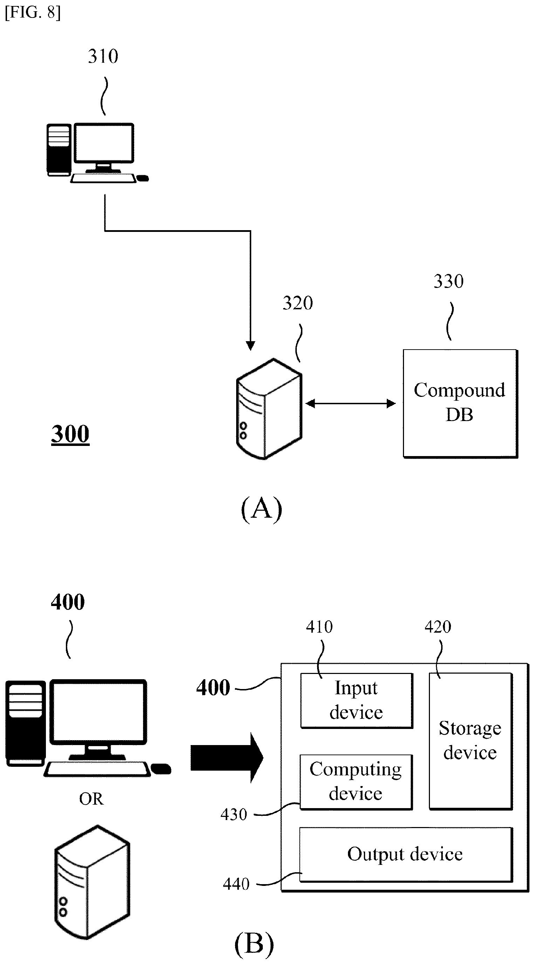

FIG. 8 illustrates an example of an apparatus for discovering a new drug candidate targeting a disorder-to-order transition region.

FIG. 8A illustrates an example of a system 300 implemented in a network. An apparatus for discovering a new drug candidate 300 includes a client device 310 and an analysis server 320. Furthermore, the apparatus for discovering a new drug candidate 300 may also include a compound DB 330. The analysis server 320 corresponds to the above-described computer device.

The client device 310 is a device which is connected to the analysis server 320 to transmit an instruction of analyzing a new drug material and receives an analysis result. In some cases, the client device 310 may provide some data required for analysis to the analysis server 320. For example, the client device 310 may provide data such as an amino acid sequence of a target protein to the analysis server 320.

The analysis server 320 uses bioinformatics to determine a disorder-to-order transition region of a target protein. The analysis server 320 uses a program (web algorithm) such as PONDR-FIT, PONDR-VLXT, and PONDR-VSL to predict a disorder region. The analysis server 320 performs molecular docking on a disorder-to-order transition region in conjunction with a library of specific compounds by executing the docking program to select first candidate compounds capable of binding to the disorder-to-order transition region from among the compound library. In addition, the analysis server 320 performs a molecular dynamics simulation on the first candidate compounds and the disorder-to-order transition region by executing a molecular dynamics simulation program to select a second candidate compound from among the first candidate compounds. Furthermore, the analysis server 320 may also perform verification on the second candidate compound as described above.

Although not illustrated in FIG. 8A, at least one of a disorder region prediction program, a docking program, and a molecular dynamics simulation program of proteins may be provided by a separate web server. In this case, the analysis server 320 accesses the corresponding web server to request execution of a task related to the corresponding program, receive the results, and performs the analysis.

The compound DB 330 may store a compound library including structural information on a specific compound, information on an amino acid sequence of a specific protein, and the like.

FIG. 8B illustrates an example of a computer device 400 for discovering a new drug candidate. The computer device 400 illustrated in FIG. 8B may also be the above-described analysis server 320. The computer device 400 refers to a device such as a personal computer (PC), a notebook-sized computer, a smart machine, or a server. The computer device 400 includes an input device 410, a computing device 420, a storage device 430, and an output device 440.

The input device 410 receives input of a user's instruction and specific data. The specific data may include at least one of an amino acid sequence of a target protein, data for bioinformatics, an amino acid sequence of a binding candidate protein, and a library of specific compounds. The input device 410 may be a communication interface device for inputting the user's instruction or specific data to the computer device 400 through communication or a separate storage device. Furthermore, the input device 410 may also be an interface device such as a keyboard, a mouse, and a touch screen.

The storage device 430 is a device which stores a docking program for performing molecular docking on a compound and a protein and a molecular dynamics simulation program of a compound and a protein specific site. Furthermore, the storage device 430 may further store at least one of an amino acid sequence of a target protein, data for bioinformatics, an amino acid sequence of a binding candidate protein, and a library of specific compounds. The storage device 430 may also store information on a candidate corresponding to the analysis result.

The storage device 430 holds a docking program for performing molecular docking on a compound and a protein and a molecular dynamics simulation program of a compound and a protein specific site. The storage device 430 uses bioinformatics to determine a disorder-to-order transition region of a target protein. The storage device 430 performs molecular docking on a disorder-to-order transition region in conjunction with a library of specific compounds by executing the docking program to select first candidate compounds capable of binding to the disorder-to-order transition region from among the compound library. In addition, the storage device 430 performs a molecular dynamics simulation on the first candidate compounds and the disorder-to-order transition region by executing a molecular dynamics simulation program to select a second candidate compound from among the first candidate compounds. Furthermore, the storage device 430 may also perform verification on the second candidate compound as described above.

The output device 440 is a device which outputs the analysis result in a fixed form. The output device 440 includes at least one of a display device, an output device for outputting a document, and a communication device for transmitting a result derived for a candidate material to another device.

Further, the method of discovering a new drug candidate as described above may be realized by a program (or application) including an executable algorithm which may be executed by a computer. The program may be stored and provided on a non-transitory computer readable medium.

The non-transitory computer readable medium does not refer to a medium storing data for a short period of time such as a register, a cache, or a memory, but refers to a medium which is capable of storing data semi-permanently and being read by an apparatus. Specifically, the above-described various applications or programs may be stored and provided on non-volatile computer readable medium such as a compact disc (CD), a digital versatile disk (DVD), a hard disk, a Blu-ray disk, a Universal Serial Bus (USB), a memory card, and a read only memory (ROM).

The present embodiments and the drawings attached to the present specification clearly illustrate only a part of the technical spirit included in the above-described technology, and it will be obvious that modified examples and specific embodiments that can be easily inferred by those skilled in the art within the scope of the technical spirit included in the specification and drawings of the above-described technology all fall within the scope of rights of the above-described technology.

## Claims

1. A method of discovering a drug candidate, the method comprising:
determining, by a computer device, a disorder-to-order transition region of a target protein using bioinformatics;
selecting, by the computer device, first candidate compounds capable of binding to the disorder-to-order transition region from among a compound library by performing molecular docking on the disorder-to-order transition region in conjunction with a library of specific compounds; and
selecting, by the computer device, a second candidate compound from among the first candidate compounds by performing a molecular dynamics simulation for the first candidate compounds and the disorder-to-order transition region.

2. The method of claim 1, wherein a molecular docking program is used, by the computer device, to perform molecular docking on any one compound and the disorder-to-order transition region comprised in the compound library.

3. The method of claim 1, wherein a compound having a higher number of sites binding to the disorder-to-order transition region or higher binding energy than reference values among the first candidate compounds is selected, by the computer device, as the second candidate compound according to the molecular dynamics simulation result.

4. The method of claim 1, further comprising confirming, by the computer device, whether the second candidate compound binds to another candidate protein,
wherein when the second candidate compound has a higher binding strength to the target protein by a reference value or more than to the other candidate protein, the second candidate compound is selected as a final candidate compound.

5. The method of claim 4, wherein the confirming whether the second candidate compound binds to another candidate protein comprises:
determining, by the computer device, a candidate region to which the second candidate protein is likely to bind based on a disorder region of the candidate protein determined based on an amino acid sequence of the candidate protein;
estimating, by the computer device, a protein structure of the candidate region based on the amino acid sequence of the candidate region; and
confirming, by the computer device, a degree to which the second candidate protein binds to a protein structure of the candidate region using a docking program.

6. The method of claim 5, wherein a protein structure of the candidate region is estimated, by the computer device, estimating a structure matched by searching the amino acid sequence of the candidate region in the Protein Data Bank (PDB) as a protein structure of the candidate region, and when there is no matched structure, the performing homology modeling based on the amino acid sequence of the candidate region.

7. A computer readable recording medium having recorded thereon a program for executing the method of discovering a new drug candidate targeting a disorder-to-order transition region described in any one of claims 1 to 6 on a computer.

8. An apparatus for discovering a drug candidate, comprising:
an input device for receiving the input of a user's instruction and specific data;
a storage device for storing a docking program for performing molecular docking on a compound and a protein and a molecular dynamics simulation program of a compound and a protein specific site; and
a computing device for using bioinformatics to determine a disorder-to-order transition region of a target protein, performing molecular docking on the disorder-to-order transition region in conjunction with a library of specific compounds by executing the docking program to select first candidate compounds capable of binding to the disorder-to-order transition region from among the compound library, and performing a molecular dynamics simulation on the first candidate compounds and the disorder-to-order transition region by executing the molecular dynamics simulation program to select a second compound from among the first candidate compounds.

9. The apparatus of claim 1, wherein the specific data comprises at least one of data for the bioinformatics and the library of specific compounds, and
the input device receives the specific data from an external storage medium or a database (DB) present in a network.

10. The apparatus of claim 1, wherein the computing device selects, as the second candidate compound, a compound having a higher number of sites binding to the disorder-to-order transition region or higher binding energy than reference values from among the first candidate compounds as a result of the molecular dynamics simulation.

11. The apparatus of claim 1, wherein the computing device confirms whether the second candidate compound also binds to another candidate protein, and selects the second candidate compound as a final candidate compound when the second candidate compound has a higher binding strength to the target protein by a reference value or more than to the other candidate protein.

12. The apparatus of claim 11, wherein the computing device determines a candidate region to which the second candidate protein is likely to bind based on a disorder region of the candidate protein determined based on an amino acid sequence of the candidate protein, estimates a protein structure of the candidate region based on the amino acid sequence of the candidate region, and uses the docking program to confirm a degree to which the second candidate protein binds to a protein structure of the candidate region.

13. The apparatus of claim 12, wherein the computing device estimates a structure matched by searching the amino acid sequence of the candidate region in the Protein Data Bank (PDB) as a protein structure of the candidate region, and when there is no matched structure, the computing device performs homology modeling based on the amino acid sequence of the candidate region to estimate a protein structure of the candidate region.
